(19) **Europäisches Patentamt**

**European Patent Office**

**Office européen des brevets**

(11) **EP 0 875 156 B1**

(12) **EUROPÄISCHE PATENTSCHRIFT**

| | |
|---|---|
| (45) Veröffentlichungstag und Bekanntmachung des Hinweises auf die Patenterteilung:<br>**16.10.2002  Patentblatt 2002/42** | (51) Int Cl.⁷: **A23L 1/30**, A23L 1/302,<br>A23L 1/304, A23L 1/0522,<br>A23L 1/0528, A23L 1/064,<br>A23L 1/10, A23L 1/16,<br>A23L 1/164, A23L 1/187,<br>A23L 1/216, A23L 1/39,<br>A23L 1/48, A23L 2/38 |
| (21) Anmeldenummer: **98106797.8** | |
| (22) Anmeldetag: **15.04.1998** | |

(54) **Produkte zur Unterstützung der Behandlung von atopischer Dermatitis**

Products to support the treament of atopic dermatitis

Proudits pour soutenir le traitement de dermatite atopique

| | |
|---|---|
| (84) Benannte Vertragsstaaten:<br>**AT BE CH DE DK FI FR GB IE LI NL SE** | (72) Erfinder: **Helling, Arnold**<br>**52355 Düren (Birgel) (DE)** |
| (30) Priorität: **16.04.1997  DE 19715878** | (74) Vertreter: **Dreiss, Fuhlendorf, Steimle & Becker**<br>**Patentanwälte**<br>**Gerokstrasse 6**<br>**70188 Stuttgart (DE)** |
| (43) Veröffentlichungstag der Anmeldung:<br>**04.11.1998  Patentblatt 1998/45** | |
| (73) Patentinhaber: **Pharmaberatung für Ernährung &**<br>**Pflege Renate Helling**<br>**52355 Düren (DE)** | (56) Entgegenhaltungen:<br>**DE-A- 2 352 797        DE-A- 4 232 140**<br>**DE-A- 4 320 526        DE-A- 19 503 993**<br>**FR-A- 2 704 390        US-A- 5 476 677**<br>**US-A- 5 480 660** |

EP 0 875 156 B1

Anmerkung: Innerhalb von neun Monaten nach der Bekanntmachung des Hinweises auf die Erteilung des europäischen Patents  kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Ernährungsprodukt zur diätetischen Unterstützung der Behandlung von atopischer Dermatitits.

Die atopische Dermatitis oder Neurodermitis atopica ist eine genetisch bedingte Stoffwechselerkrankung, die durch eine IgE-vermittelte Überempfindlichkeitsreaktion vom Sofort-Typ I bis IV sowie weitere immunologische und nicht immunologische Faktoren bedingt ist. Die Haut ist wegen einer Unterfunktion der Talg- und Schweißdrüsen glanzlos und trocken. Die damit verbundenen Hauptsymptome sind ein quälender Juckreiz sowie Hautekzeme. Sehr oft geht die atopische Dermatitis mit einer Überempfindlichkeit gegen bestimmte Nahrungsmittel einher, da zahlreiche Stoffwechselwege gestört sind.

Neben der medikamentösen Behandlung der atopischen Dermatitis achten die Betroffen auch auf eine angemessene allergenarme Ernährung. Dies ist besonders schwierig, weil bei jedem einzelnen Produkt auf die Inhaltsstoffe und ggf. persönliche Unverträglichkeiten oder allergische Reaktionen geachtet werden muss. Dies wird dadurch noch erschwert, dass in handelsüblichen Produkten u.a. oft versteckte Allergene enthalten sind, die nach geltendem Lebensmittelrecht nicht deklariert werden müssen (Beispiel: Eipulver). Eine allergiebedingte Lebensmittelauswahl bzw. Lebensmitteleinschränkung führt zu einer einseitigen Ernährung und auch häufig zu Mangelerscheinungen.

Von allen an atopischer Dermatitis erkrankten Personen sind ca. 40 % Kinder bis zum 11. Lebensjahr. Insbesondere für Kinder ist die Krankheit besonders quälend, weil sie besonders auf ihre Hautpflege achten und oft strenge Diät halten müssen, die in der heutigen Form zu Mangelernährung führen kann.

Aus der US 5,476,677 ist bekannt geworden, Cerealien derart zu präparieren und chemisch aufzubereiten, dass sie weniger Allergene aufweisen.

Aus der US 5,480,660 ist bekannt geworden, aus Weizen allergene Proteine abzuscheiden und so einen hypoallergenen, tierischen Rohstoff bereitzustellen.

Aufgabe der Erfindung ist es, Ernährungsprodukte zur Unterstützung der Behandlung von atopischer Dermatitits anzubieten, die einen Großteil der Grundversorgung und eine vielseitige Ernährung ohne Mangelerscheinungen gewährleisten und die auch kindgerecht sind.

Die Lösung besteht in einem Produkt mit den Merkmalen des Anspruchs 1.

Wesentlich an den erfindungsgemäßen Produkten ist, dass sie weitgehend auf hypoallergenen Rohstoffen basieren, die zusätzlich noch hydrolysiert, d.h. aufgeschlossen sein können, und Zusatzstoffe zum Ausgleich der Stoffwechselstörungen enthalten. Unter hautverbessernden Substanzen werden solche Stoffe verstanden, die Ausschläge oder Ekzeme lindern bzw. deren Entstehung hindern und die den Juckreiz lindern. Ein zeitraubender Vergleich der Inhaltsstoffe beim Kauf handelsüblicher Produkte entfällt daher. Der Gefahr von Mangelerscheinungen wird vorgebeugt. Die erfindungsgemäßen Wirkstoffkombinationen gewährleisten eine ausreichende Versorgung mit diätetisch wirksamen Produkten. Mit den erfindungsgemäßen Produkten ist eine weitgehende Grundversorgung bei der Ernährung möglich, ohne auf handelsübliche Produkte zurückgreifen zu müssen, die u.a. versteckte Allergene enthalten können.

Besonders vorteilhaft ist bei den Ernährungsprodukten die Versorgung mit Gamma-Linolensäure und Omega-3-Fettsäuren. Dies gleicht die bei Atopikern häufiger zu beobachtenden Störungen im Fettstoffwechsel aus, die mit einem Gamma-Linolensäure-Mangel einhergehen und eine der Ursachen des Juckreizes sind.

Erfindungsgemäß kann vorgesehen sein, dass das Ernährungsprodukt Reismilchpulver enthält, das 10 - 80 Teile Stärke, vorzugsweise Kartoffelstärke, als Trägermaterial enthält.

Ferner ist erfindungsgemäß denkbar, dass als Gamma-Linolensäurehaltige Stoffe Borretschsamenöl und/oder schwarze Johannesbeerkerne enthalten sind.

Eine weitere Ausführungsform der Erfindung sieht vor, dass als Omega-3-Fettsäuren enthaltende Stoffe Leinsamen, Leinöl, schwarze Johannesbeerkerne, Fischöl, grünes Blattgemüse bzw. deren Extrakte, enthalten sind.

Bei einer weiteren Ausgestaltung der Erfindung findet getrocknetes Reismilchpulver, das mit Gamma-Linolensäure und Omega-3-Fettsäuren angereichert ist, Verwendung.

Es kann auch vorgesehen sein, dass das Ernährungsprodukt als Zusatzstoff wenigstens eine Darmflora und/oder Darmschleimhaut kräftigende Substanz enthält.

Ferner ist erfindungsgemäß denkbar, dass als Darmflora und/oder Darmschleimhaut kräftigende Substanz Inulin und/oder Oligofructose und/oder Apfelpektin und/oder Guar und/oder Carob enthalten ist oder sind.

Eine weitere Ausführungsform der Erfindung sieht vor, dass als Darmflora und/oder Darmschleimhaut kräftigende Substanz Amaranth bzw. Kartoffeln enthalten ist oder sind.

Erfindungsgemäß kann vorgesehen sein, dass ferner Spurenelemente, Jodsalz, Mineralstoffe wie Calcium, Magnesium oder Zink bzw. mineralstoffhaltige Substanzen wie Zinkhefe, Aromen und/oder Hilfsstoffe wie Backpulver oder Zitronensäure enthalten sind.

Unter den erfindungsgemäßen Ernährungsprodukten befinden sich insbesondere Lebensmittel für die Zufuhr komplex gebundener Kohlenhydrate, z.B. Knabberkekse, Brötchen, Pfannkuchen, Waffeln, Pizzas, Kartoffelpüree, Flakes, Nudeln, Lachs-Sauce, entweder als Fertigprodukte oder als Teig- bzw. Pulvermischungen, die nur mit Wasser, jedoch nicht mit Milch, angerührt und gebacken/gekocht werden.

Als hypoallergener Weizenersatzstoff für solche Lebensmittel eignet sich insbesondere Kamut, in Anteilen zwischen 30 % und 90 %. Amaranth und Kartoffeln weisen einen hohen Gehalt an Glutamin bzw. dessen Vorläufer auf Amaranth kann in Anteilen von 5 bis 25 % eingesetzt werden.

Unter den erfindungsgemäßen Ernährungsprodukten befinden sich ebenfalls Getränke, wie z.B. Reismilch, Cola-Getränke, Orangen-Zitronen-Drinks, entweder als Instant-Pulver zum anrühren mit Wasser oder als Fertiggetränk portioniert. Diese Getränke bzw. Getränkepulver enthalten bevorzugt Reismilch bzw. Reismilchpulver als hypoallergene Basissubstanz in Mengen von 40 % bis 95 %.

Unter den erfindungsgemäßen Ernährungsprodukten findet man ebenfalls sogenannte Süßigkeiten wie Eis, Pudding, Fruchtaufstrich und Reismilch-Snack. Auch hier wird bevorzugt Reismilch bzw. Reismilchpulver in Mengen von 40 % bis 95 % eingearbeitet.

Das erfindungsgemäße Reismilch-Pulver enthält Reis und Kartoffelstärke als Trägersubstanz, bevorzugt hydrolisierte Kartoffelstärke (Kartoffelmaltodextrin), da diese eine bessere Löslichkeit hat, ist sprühgetrocknet und instantisiert. Es kann als Instantpulver angeboten werden, welches mit Wasser angerührt werden kann. Es kann ferner als fertiges Milchersatzgetränk in Tüten oder Flaschen angeboten werden. Es wird ebenfalls als Basissubstanz bei der Herstellung anderer erfindungsgemäßer Ernährungsprodukte verwendet.

Mit den erfindungsgemäßen Ernährungsprodukten soll wie erwähnt auch der Gefahr einer Mangelernährung vorgebeugt werden, die mit einer insbesondere für Atopiker notwendigen diätetischen Einschränkung auf einige wenige erlaubte Lebensmittel einhergehen kann. Die erfindungsgemäßen Ernährungsprodukte sind daher bevorzugt mit Vitaminen, Mineralstoffen und Spurenelementen angereichert.

Insbesondere kann eine ausgewogene Anreicherung in solcher Weise erfolgen, daß der tägliche Bedarf an diesen Stoffen durch das Verzehren von 3 bis 6 Portionen frei ausgewählten erfindungsgemäßen Ernährungsprodukte gedeckt wird.

Eine Kombination von

| Vitamin C | 15,0 mg |
| Niacin | 4,5 mg |
| Vitamin E | 2,50 mg |
| B-Carotin | 2,00 mg |
| Pantothensäure | 1,5 mg |
| Vitamin B6 | 0,50 mg |
| Vitamin B2 | 0,40 mg |
| Vitamin B1 | 0,35 mg |
| Folsäure | 0,05 mg |
| Biotin | 0,0375 mg |
| Vitamin B12 | 0,25 ug |

in einer Portion Ernährungsprodukt deckt 25 % des durchschnittlichen Tagesbedarfes an diesen Stoffen ab.

Weitere vorteilhafte Weiterbildungen ergeben sich aus den Unteransprüchen.

Im folgenden werden die erfindungsgemäßen Ernährungsprodukte aufgrund von Ausführungsbeispielen näher beschrieben.

1. Reismilch-Pulver als diätetisches Lebensmittel

Zur Herstellung des Instantpulvers wird zunächst Reismilch in konzentrierter Form hergestellt (Eindickungsquote ca. 10 %), anschließend sprühgetrocknet und teilinstantisiert.

Das Prinzip der Sprühtrocknung beruht darauf, dass in einem mit heißer Luft durchströmten Raum das zu trocknende Gut fein versprüht bzw. vernebelt wird. Hierzu können z.B. Zerstäuberscheiben oder Düsen verwendet werden. Das Versprühen führt zu einer Vergrößerung der Oberfläche des zu trocknenden Gutes ( 1 Liter Flüssigkeit entspricht ca. 120 m2), wodurch ein schneller Wärmeübergang gewährleistet ist und das Produkt relativ schonend zu trocknen ist. Die Temperatur der heißen Luft liegt vorzugsweise bei 170 - 200°C. Das Trockengut erreicht dabei eine maximale Temperatur von ca. 60 - 80°C, bedingt durch die Bildung einer Dampfhülle (Leidenfrostsches Phänomen).

Bei der Sprühtrocknung der Reismilch bedarf es eines Trägermaterials, um ausreichend gute Trocknungseigenschaften zu bekommen. Geeignet ist z.B. Stärke. Als gut verträgliche allergenarme Trägersubstanz wird vorzugsweise Kartoffelstärke verwendet. Der Anteil beträgt vorteilhafterweise etwa 10 - 50 % der Gesamtmenge, kann jedoch auch darüber liegen.

Die Kartoffelstärke wird in der Reismilch gelöst. Die Lösung wird zur Abtötung von Keimen erhitzt. Die mikrobiologisch saubere Lösung wird wie beschrieben sprühgetrocknet.

Bei der Sprühtrocknung entsteht ein Feinpulver, dass aus der Abluft entfernt und dem getrockneten Gut wieder zugeführt wird. Beim Teilinstantisieren wird dieses Feinpulver grundsätzlich angefeuchtet, agglomeriert und nachgetrocknet. Z.B. kann es im Bereich der Zerstäuberscheibe wieder in den Trockenturm geleitet werden, so dass es sich an das noch flüssige Trockengut anhaftet. So entstehen Agglomerate, die sich besser benetzen lassen und so die Löslichkeit des Endprodukts verbessern.

Reismilch ist ein hypoallergener Rohstoff mit einer milden Süße, bedingt durch den Anteil an Reis-Kohlehydraten. Ein Zuckerzusatz ist nicht notwendig. Es dient als Ersatz für tierische Milchprodukte, Milchprodukte aus Nüssen und Sojaprodukte. Es kann ferner in natürlicher oder angereichter Form als Basis für zahlreiche andere erfindungsgemäße Ernährungsprodukte dienen.

Das erfindungsgemäße diätetische Lebensmittel hat die folgende Rezeptur

| Inhaltsstoffe | Teile |
|---|---|
| Reismilchpulver, sprühgetrocknet auf Trägersubstanz Kartoffelstärke | 90,5 |
| Inulin | 2 |
| Borretschsamenöl | 2,5 |
| Vitamine, Aromen, Jodsalz, Calciumcarbonat | 5 |

Das Reismilchpulver ist mit Gamma-Linolensäure aus Borretschsamenöl angereichert. 150 ml Reismilch enthalten bei der oben genannten Rezeptur ca. 50 mg Gamma-Linolensäure. An Vitaminen sind die Vitamine E, Beta-Karotin, die Vitamine B1, B2, B6, B12, Niacin, Pantothensäure, Biotin und Folsäure zugesetzt. Die Dosierung pro 150 ml entspricht einem Viertel des Tagesbedarfs nach RDA.

Variationen der Geschmacksrichtungen, z.B. Natur oder "Schokolade" (mit Carob, d.h. Johannisbrotmehl) sind möglich.

Gamma-Linolensäure, Omega-3-Fettsäuren lindern den Juckreiz. Durch Inulin bzw. Oligofructose wird die physiologische Darmflora gekräftigt. Johannisbrotmehl (Carob) zeichnet sich durch einen hohen Anteil an löslichen Faserstoffen aus, die zu kurzkettigen Fettsäuren abgebaut werden, welche sich günstig auf die Funktion der Dickdarmschleimhaut auswirken.

2. Pudding-Pulver

| Inhaltsstoffe | Teile |
|---|---|
| Reismilchpulver, sprühgetrocknet auf Trägersubstanz Kartoffelstärke | 86 |
| Schw. Johannisbeerkerne | 2 |
| Kartoffelstärke | 6 |
| Inulin | 1 |
| Jodsalz, Vitamin E, Aromen, Calciumcarbonat | 3 |
| Borretschsamenöl | 2 |

Im Unterschied zum soeben beschriebenen Reismilchpulver enthält das Puddingpulver noch einen Anteil an Kartoffelstärke, um die für Pudding typische Konsistenz zu erzielen. Es sind mehrere Geschmacksrichtungen wie "Schokolade" (mit Carob, d.h. Johannisbrotmehl) oder Mandel (mit Mandelaroma) denkbar. Die Zubereitung kann mit Leinöl erfolgen, um den Juckreiz zu vermindern.

Das Puddingpulver muss nur noch mit Wasser angerührt werden, da es im wesentlichen aus Reismilchpulver besteht. Das Hinzufügen von häufig allergieauslösenden Milchprodukten (z.B. Kuhmilch, Sojamilch) entfällt somit. Das Produkt kann ebenfalls als Fertigprodukt im Handel angeboten werden. Bei der industriellen Herstellung als Fertigprodukt wird vorteilhaft ein Teil des Reismilchpulvers durch flüssige Reismilch ersetzt.

3. Eis (Eispulver)

| Inhaltsstoffe | Teile |
|---|---|
| Reismilchpulver, sprühgetrocknet auf Trägersubstanz Kartoffelstärke | 77,5 |
| Schw. Johannisbeerkerne | 2 |
| Sonnenblumenöl | 4 |
| Leinöl | 4 |
| Borretschsamenöl | 1 |
| Inulin | 1 |
| Aromen und Calciumcarbonat | 8,5 |
| Vitamine, Zitronensäure | 2 |

Im Vergleich zu dem unter (1) beschriebenen Reismilch-Pulver ist die Dosierung an Gamma-Linolensäure durch schwarze Johannisbeerkerne und Leinöl auf ca. 100 mg pro 100 g Fertigeis erhöht. Die Vitamindosierungen entsprechen einem halben Tagesbedarf nach RDA pro 100 g Fertigeis. Verschiedene Geschmacksrichtungen wie z. B. "Schokolade" (mit Carob, d.h. Johannisbrotmehl) oder Papaya sind möglich. Eine mögliche Zugabe von Magnesium unterstützt den juckreizlindernden Effekt. Mit der Zugabe von Calcium wird dem durch den Verzicht auf tierische Milchprodukte möglichen Calciummangel vorgebeugt. Durch Inulin oder Oligofructose wird die Darmflora gekräftigt.

Die Rezeptur kann als fertiges Eis (Eis am Stiel, Eis im Hörnchen, Eis im Becher, vgl. unten Beispiel 6) angeboten werden. Sie kann ferner als pulverförmige Mischung angeboten werden, die mit Wasser angerührt z.B. in einer Haushalts-Eismaschine zu Eis verarbeitet wird.

4. Knabberkekse

| Inhaltsstoffe | Teile |
|---|---|
| Kamut | 65,4 |
| Schw. Johannisbeerkerne | 4 |
| Amaranth | 10 |
| Reismehl | 4 |
| Kartoffelflocken | 5 |
| Leinöl | 4 |
| Inulin | 2 |
| Borretschsamenöl | 2,5 |
| Natriumbicarbonat, Eisen-II-gluconat, Vitamine, Jodsalz | 3,1 |

In diesem Produkt sind die Gehalte an Gamma-Linolensäure und Omega-3-Fettsäuren durch den Anteil an schwarzen Johannisbeerkernen, Leinöl und Borretschsamenöl jeweils etwa 250 mg und 2,3 g pro 100 g Kekse. Als Vitamine sind Vitamine E, C, die Vitamine B1, B2, B6, B12, Biotin, Niacin, Pantothensäure, Beta-Carotin und Folsäure zugesetzt. Die Vitamindosierung liegt bei 100 bis 150 % des Tagesbedarfs pro 100 g. Inulin bzw. Oligofructose wirkt sich positiv auf die Darmberuhigung aus.

Dieses erfindungsgemäße Ernährungsprodukt enthält als allergenarme Rohstoffe Kamut, Amaranth, Kartoffelflocken und Reismehl. Amaranth ist reich an Magnesium, das die Neigung zu Juckreiz verringert. Amaranth und Kartoffeln weisen ferner einen hohen Gehalt an Glutamin bzw. Glutaminvorläufersubstanzen auf, die sich günstig auf die Dickdarmschleimhaut auswirken. Durch den Zusatz von Vitamin B6 ist gewährleistet, dass Glutamin oder dessen Vorläufersubstanzen gut verträglich sind. Natriumbicarbonat dient als Treibmittel.

Vorteilhaft ist ferner der hohe Lysingehalt von Amaranth. Lysin mindert die Heftigkeit neurodermitischer Hautreaktionen. Der Rohstoff Amaranth zeichnet sich auch durch einen hohen natürlichen Calcium- und Eisengehalt aus. Damit wird Mangelerscheinungen, die durch eine einseitige Diät bedingt sein können, vorgebeugt.

5. Brötchen

| Inhaltsstoffe | Teile |
|---|---|
| Kamut (ausgesiebt) | 61 |
| Amaranth | 16,5 |
| Schw. Johannisbeerkerne | 2 |
| Reismehl | 4 |
| Leinöl | 4 |
| Sonnenblumenöl | 4 |
| Natriumbicarbonat, Calciumcarbonat, Jodsalz, Vitamin E, Sauerteig | 5,5 |
| Inulin | 2 |
| Borretschsamenöl | 1 |

Das erfindungsgemäße Produkt kann als tiefgekühlte, fertig gebackene Brötchen oder als Teigmischung zum Selberbacken mit Wasser bzw. Leinöl angeboten werden. Vorteilhaft ist der Zusatz einer natürlich gewachsenen Zinkhefe, da bei einer milchproduktlosen Ernährung auch Mangelsituationen bei Zink möglich sind. Der Sauerteig senkt den Gehalt an allergener Phytinsäure, die normalerweise die Resoption von Spurenelementen im Darm hemmt. So ist eine gute Spurenelementaufnahme möglich. Das erfindungsgemäße Produkt zeichnet sich ferner durch die Verwendung hypoallergener Rohstoffe wie Kamut und Amaranth aus.

6. Pfannkuchen- bzw. Waffelteigmischung

| Inhaltsstoffe | Teile |
|---|---|
| Kamut | 54,6 |
| Reismilchpulver, sprühgetrocknet auf Trägersubstanz Kartoffelstärke | 25 |
| Amaranth | 10 |
| Schw. Johannisbeerkerne | 1 |
| Inulin | 2 |
| Borretschsamenöl | 0,4 |
| Natriumbicarbonat, Calciumcarbonat, Vitamin E, Jodsalz | 7 |

Für dieses Produkt gelten die bisherigen Erläuterungen analog. Die Vitamine sind dieselben wie im Reismilchpulver nach (1).

7. Fruchtaufstrich

| Inhaltsstoffe | Teile |
|---|---|
| Kirschen | 80 |
| Schw. Johannisbeerkerne | 2 |
| Fruchtzucker | 5 |
| Zitronensäure | 4 |
| Inulin | 2 |
| Vitamine, Eisen-II-gluconat | 3 |
| Borretschsamenöl | 1 |
| Leinöl | 3 |

Der erfindungsgemäße Fruchtaufstrich zeichnet sich durch die Verwendung von Kirsch und Fruchtzucker als hypoallergene Rohstoffe aus. Die Vitaminzugabe entspricht derjenigen des Reismilchpulver nach (1) zzgl. Vitamin C.

8. Pizza

| Inhaltsstoffe | Teile |
|---|---|
| Kamut (ausgemahlen) | 48,2 |
| Amaranth | 10 |
| Reismehl | 4 |
| Champignons, Kartoffeln, Zuchini | 14 |
| Schw. Johannisbeerkerne | 2,5 |
| Inulin | 2 |
| Leinöl | 4 |
| Jodsalz, Calciumcarbont, Natriumcarbonat Vitamin E | 11 |
| Borretschsamenöl | 0,3 |
| Sonnenblumenöl | 4 |

Das Produkt kann als tiefgekühlte oder vakuumverpackte fertige Pizza angeboten werden. Es kann aber auch lediglich der tiefgekühlte oder vakuumverpackte Pizzaboden oder eine Backmischung angeboten werden, die mit Wasser bzw. Leinöl zubereitet werden kann. Der Boden kann ferner mit Pilzen und anderen hypoallergenen bzw. individuell gut verträglichen Produkten belegt werden. Zu den Inhaltsstoffen des Pizzabodens wird auf die obigen Ausführungen verwiesen.

9. Kartoffelpüree

| Inhaltsstoffe | Teile |
|---|---|
| Kartoffelflocken | 75 |
| Reismilchpulver (sprühgetrocknet) auf Trägersubstanz Kartoffelstärke | 22,5 |
| Inulin | 2 |
| Borretschsamenöl | 0,5 |

Diese Mischung kann in Portionsbeutel abgefüllt angeboten werden. Dieses Produkt muss nicht mit Milch, die für Personen mit atopischer Dermatitis meist unverträglich ist, angerührt werden, da Reismilchpulver (als Milchersatz) schon enthalten ist. Es genügt die Zugabe von Wasser und eventuell etwas Leinöl. Zu den Inhaltsstoffen wird auf die obigen Ausführungen verwiesen.

10. Nudeln

| Inhaltsstoffe | Teile |
|---|---|
| Kamut (ausgemahlen) | 86,8 |
| Schw. Johannisbeerkerne | 1 |
| Amaranth | 10 |
| Inulin | 1 |
| Borretschsamenöl | 1,2 |

Nudeln (vor allem in phantasievollen, spielerischen Formen) sind vor allem bei Kindern sehr beliebt. Diese brauchen nicht auf ihre Lieblingspeise zu verzichten, da diese auf dem hypoallergenen Weizenersatzstoff Kamut basiert. Auf Vitaminzusätze kann hier verzichtet werden, weil diese beim Kochen verloren gehen würden.

11. Lachs-Sauce

| Inhaltsstoffe | Teile |
|---|---|
| Reismilchpulver (sprühgetrocknet) auf Trägersubstanz Kartoffelstärke | 48 |
| getrockneter Lachs | 26 |
| Fischöl | 10 |
| Inulin | 4 |
| Borretschsamenöl | 4 |
| Zitronensäure | 2 |
| Jodsalz, Aromen | 6 |

Das Produkt kann als in Portionsbeutel abgefülltes Saucenpulver angeboten und mit Wasser und evtl. etwas Leinöl zubereitet werden. Fischöl enthält wie das Leinöl ebenfalls Omega-3-Fettsäuren, die dem Juckreiz entgegenwirken.

12. Frühstücksflocken

| Inhaltsstoffe | Teile |
|---|---|
| Reis (gemahlen) | 67,9 |
| schwarze Johannesbeerkerne | 2 |
| Amaranth | 15 |
| Topinambur | 6 |
| Calciumcarbonat, Eisen-II-gluconat Magnesiumcarbonat, Vitamine, Zitronensäure | 4,3 |
| Apfelpektin | 0,9 |
| Borretschsamenöl | 0,9 |
| Leinöl | 3 |

Die Frühstücksflocken enthalten nur allergenarme Rohstoffe, nähmlich Reis, Amaranth, Topinambur. Apfelpektin versorgt die Dickdarmschleimhäute mit kurzkettigen Fettsäuren. Die Vitaminzusätze entsprechen denjenigen beim Reismilchpulver nach (l) zzgl. Vitamin C.

13. Cola-Getränk, als Instant-Pulver

| Inhaltsstoffe | Teile |
|---|---|
| Reismilchpulver (sprühgetrocknet) auf Trägersubstanz Kartoffelstärke | 80 |
| schwarze Johannisbeerkerne | 6 |
| Zitronensäure | 6 |
| Aromen, Magnesiumcarbonat, Calciumcarbonat, Vitamine | 6 |
| Inulin u/o Oligofructose | 1 |
| Borretschsamenöl | 1 |

Hinsichtlich der Inhaltsstofe gelten die bisherigen Ausführungen. Die Vitaminzugabe entspricht derjenigen bei der Reismilchpulver nach (1).

14. Cola Getränk, als Fertiggetränk

| Inhaltsstoffe | Teile |
|---|---|
| Reismilch | 55 |
| Fruchtzucker | 16 |
| Aromen, Vitamine, Calcium und Magnesiumcarbonat, Emulgator | 16 |
| Zitronensäure | 8 |
| Borretschsamenöl | 3 |
| Leinöl | 1 |
| Inulin | 1 |

15. Orangen-Zitronen-Getränk, als Instant-Pulver

| Inhaltsstoffe | Teile |
|---|---|
| Reismilchpulver (sprühgetrocknet) auf Trägersubstanz Kartoffelstärke | 80 |
| schwarze Johannesbeerkerne | 6 |
| Zitronensäure | 6 |
| Aromen, Magnesiumcarbonat, Calciumcarbonat, Vitamine | 6 |
| Inulin u/o Oligofructose | 1 |
| Borretschsamenöl | 1 |

Auch hier gelten die bisherigen Erläuterungen. Die Vitaminzusätze entsprechen denjenigen des Reismilchpulvers nach (1).

16. Orangen-Ztronen Fertiggetränk

| Inhaltsstoffe | Teile |
|---|---|
| Reismilch | 55 |
| Fruchtzucker | 16 |
| Aromen, Vitamine, Calcium und Magnesiumcarbonat, Emulgartor | 16 |
| Zitronensäure | 8 |
| Borretschsamenöl | 3 |
| Leinöl | 1 |
| Inulin | 1 |

Auch hier gelten die bisherigen Erläuterungen.

17. Reismilch-Snack

| Inhaltsstoffe | Teile |
|---|---|
| Reismilch | 53 |
| Kamut | 15 |
| Sonnenblumenöl | 12 |
| Fruchtzucker | 6 |

(fortgesetzt)

| Inhaltsstoffe | Teile |
|---|---|
| Aromen, Vitamine, Calcium und Magnesiumcarbonat | 5 |
| Leinöl | 3 |
| Schw. Johannesbeerkerne | 2 |
| Zitronensäure | 2 |
| Borretschsamenöl | 1 |
| Inulin | 1 |

Auch hier gelten die bisherigen Erläuterungen.

Den Gehalt an Omega-3-Fettsäuren der oben als Beispiele aufgeführten erfindungsgemäßen Produkte, pro fertig angerichteter Portion, ist in der Tabelle 1 zusammengefasst.

Tabelle 1

| Produkt | pro Portion Omega-3-Fettsäuren in g | Fertigprodukt Portionsgröße | Energiewert in kcal pro Portion |
|---|---|---|---|
| Reismilch | 0,5 | 150 ml | 98 |
| Pudding | 0,9 | 150 g | 104 |
| Eis | 1,1 | 50 g | 163 |
| Knabberkekse | 0,4 | 20 g | 72 |
| Brötchen | 1,2 | 50 g | 182 |
| Waffelteig | 1,9 | 100 g | 498 |
| Fruchtaufstrich | 0,8 | 50 g | 39 |
| Pizza | 1,9 | 80 g | 280 |
| Kartoffelpüree | 1,8 | 100 g | 401 |
| Nudeln | 0,01 | 50 g | 176 |
| Lachs-Nudelsauce | 0,9 | 100 g | 38 |
| Flakes | 0,5 | 30 g | 92 |
| Cola | 0,1 | 200 ml | 59 |
| Orangen-Zitronen-Getränk | 0,1 | 200 ml | 59 |
| Reismilch-Snack | 0,8 | 30 g | 118 |

Der Gehalt an Gamma-Linolensäure pro Portion und Produkt liegt immer bei 50 mg.

Diese erfindungsgemäßen Einzelzubereitungen stehen beispielhaft für die Wirkung des erfindungsgemäßen Ernährungsprodukts. Vorhandene Stoffwechselstörungen und Ungleichgewichte werden ausgeglichen, der Juckreiz wird gemindert, der Verdauungstrakt wird gekräftigt, der Energie-Grundbedarf wird unter Berücksichtigung persönlicher Vorlieben weitgehend abgedeckt. Weitere Grundnahrungsmittel, z.B. Obst und Gemüse, sollten je nach Verträglichkeit in den Ernährungsplan aufgenommen werden. Die erfindungsgemäßen Produkte beeinflussen z.B. die Versorgung mit Gamma-Linolensäure zum Ausgleich von Stoffwechselstörungen im Bereich der Omega-6-Fettsäuren. Die täglich aufzunehmende Menge an Gamma-Linolensäure sollte 250 mg und mehr betragen. Die erfindungsgemäßen Produkte decken pro Produkt und Portion den Bedarf an Gamma-Linolensäure zu 20 % ab, so dass täglich die Mindestmenge von 250 mg Gamma-Linolensäure erreicht wird bei vorwiegender oder ausschließlicher Ernährung mit den erfinderischen Ernährungsprodukten. Dies wird durch die, in der beigefügten Tabelle aufgestellten Modellrechnungen nochmals deutlich.

Tabelle 2

| Beispielrechnungen der täglichen Aufnahme von Omega-3-Fettsäuren und Gamma-Linolensäure | | |
|---|---|---|
| Beispielrechnung 1 | pro Portion Omega-3-Fettsäuren in g | pro Portion Gamma-Linolensäure in mg |
| Reismilch | 0,5 | 50 |

Tabelle 2   (fortgesetzt)

| Beispielrechnung 1 | pro Portion Omega-3-Fettsäuren in g | pro Portion Gamma-Linolensäure in mg |
|---|---|---|
| Flakes | 0,5 | 50 |
| Kartoffelpüree | 1,8 | 50 |
| Waffelteig | 1,9 | 50 |
| Orangen-Zitronen-Getränk | 0,1 | 50 |
|  | **4,8 g** | **250 mg** |
| **Beispielrechnung 2** | **pro Portion Omega-3-Fettsäuren in g** | **pro Portion Gamma-Linolensäure in mg** |
| Reismilch | 0,5 | 50 |
| Nudeln | 0,01 | 50 |
| Lachs-Nudelsauce | 0,9 | 50 |
| Brötchen | 1,2 | 50 |
| Cola | 0,1 | 50 |
|  | **2,7 g** | **250 mg** |

Die erfindungsgemäßen Produkte wirken ferner der bei Atopikern oft gesteigerten Histaminbildung entgegen und wirken somit juckreizmindernd. Dies wird dadurch erreicht, dass die erfindungsgemäß gewählten hypoallergenen Rohstoffe einen geringen Histaminfreisetzungsgrad aufweisen und dass die erfindungsgemäßen Produkte ausreichend mit Folsäure (pro Portion 25 % des Tagesbedarfs nach RDA) angereichert sind, und einen hohen Gehalt an Magnesium und Omega-3-Fettsäuren in Form von Leinöl, Fischölen, schwarze Johannesbeerkernen oder Borretschsamenöl aufweisen. Als Lieferant von Omega-3-Fettsäuren können aber auch grünes Blattgemüse oder Extrakte daraus dienen.

Bei Fischöl ist darauf zu achten, dass nur gasdichte Verpackungsfolien benutzt werden und die Abfüllung unter Sauerstoffaustausch geschieht, damit der Oxidationsprozess eingeschränkt ist. Leinöl wird vorteilhafterweise bei der frischen Zubereitung von Produkten wie z.B. Salat im Austausch zu Olivenöl oder Sonnenblumenöl angewendet. Ferner kann es bei tiefgefrorenen Produkten angewendet werden, da es hierbei nicht oxidiert.

Die schwarzen Johannesbeerkerne werden vorzugsweise in leicht gemahlener Form in die erfindungsgemäßen Produkte eingebracht, damit die Inhaltstoffe für den Körper besser verwertbar sind. Der Anteil an Omega-3-Fettsäuren ist ausreichend hoch dosiert, so dass bei regelmäßiger Verwendung der Produkte, z.B. analog der Beispielrechnungen, eine Tagesdosis von mindestens l g erreicht wird.

Da bei der Aufnahme von Omega-3-Fettsäuren als Oxidationsschutz zusätzliches Vitamin E vorteilhaft ist, sind die erfindungsgemäßen Produkte mit Vitamin E angereichert.

Die körpereigene Darmflora wird durch Zugabe von Inulin bzw. Oligofructose gekräftigt. Durch die Verwendung glutaminhaltiger Rohstoffe wie Kamut, Amaranth und Kartoffeln sowie von Rohstoffen mit wasserlöslichen Faserstoffen wie Apfelpektin, Guar und Carob werden die körpereigenenen Darmschleimhäute stabilisiert.

Vorteilhaft ist auch die Auswahl allergenarmer Rohstoffe und der Zusatz von Vitaminen, Spurenelementen und Mineralstoffen. Hierbei sollten pro Portion 10 - 50 %, vorteilhafterweise 25 % des Tagesbedarfs nach RDA enthalten sein. Die Rohstoffe enthalten vorteilhafterweise weitgehend keine biogenen Amine, phototoxischen Stoffe oder andere Stoffe mit allergischem oder pseudoallergischem Potential. Die bevorzugten Haupt-Rohstoffe sind Borretschsamenöl, schwarze Johannesbeerkerne, Leinöl, Reis, Reismilchpulver, Fischöl, Kamut, Amaranth, Kartoffeln, Kartoffelflocken. Borretschsamenöl enthält ca. 20 % Gamma-Linolensäure und ermöglicht somit, durch Aufnahme mehrerer erfindungsgemäßer Portionen, eine ausreichende tägliche Aufnahmemenge von bis über 500 mg durch die erfindungsgemäßen Produkte. Schwarze Johannisbeerkerne und Leinöl enthalten, neben kleineren Anteilen an Gamma-Linolensäure, Omega-3-Fettsäuren. Kamut, Amaranth und Kartoffel sind hypoallergene, hochwertige Rohstoffe mit hohem Eiweiss-Mineralstoff- und Spurenelementgehalt.

Reismilch dient als Ersatz für tierische Milch- und Sojamilchprodukte. Das erfindungsgemäße sprühgetrocknete Instantpulver bewirkt, dass Reismilch als hypoallergener Rohstoff in einer Vielzahl von Ernährungsprodukten eingesetzt werden kann. Das Pulver oder Fertiggetränk kann auch überall hin mitgenommen werden und erhöht somit die Mobilität insbesondere von Kindern mit atopischer Dermatitis.

Bei den erfindungsgemäßen Produkten wird die Grundversorgung an Mahlzeiten und Getränken unter Berücksichtigung persönlicher Vorlieben weitgehend abgedeckt. Die Produkte können über den ganzen Tag verteilt eingesetzt werden.

Die erfindungsgemäßen Ernährungsprodukte dienen als unterstützende, diätetische Maßnahme einer ärztlichen Behandlung der atopischen Dermatitis. Die therapeutische Behandlung kann sich über Jahre hinziehen.

Da erwiesenermaßen eine allergenarme Ernährung der stillenden Mutter die Atopierate beim Säugling um mehr als die Hälfte senken kann, sind die erfindungsgemäßen Ernährungsprodukte auch für alle stillenden Mütter empfehlenswert. Bei genetisch bedingter Anlage eines Elternteils oder wenn innerhalb der Familie bereits atopische Dermatitis aufgetreten ist, wird durch die Verwendung der erfindungsgemäßen Ernährungsprodukte während der Stillzeit das Risiko des Auftretens von atopischer Dermatitis beim Säugling verringert.

## Patentansprüche

1. Ernährungsprodukt zur diätetischen Unterstützung der Behandlung von atopischer Dermatitis, wobei seine Basissubstanzen aus hypoallergenen Rohstoffen bestehen, **dadurch gekennzeichnet, dass** es als Zusatzstoffe wenigstens einen gammalinolensäurehaltigen Stoff und einen Omega-3-Fettsäuren enthaltenden Stoff enthält, wobei die Zusatzstoffkombination zum Ausgleich der Stoffwechselstörungen bei atopischen Dermatitis und/oder zur Juckreizverminderung beiträgt.

2. Ernährungsprodukt nach Anspruch 1, **dadurch gekennzeichnet, dass** als hypoallergene Rohstoffe getrocknetes Reismilchpulver und/oder Kamut und/oder Amaranth und/oder Reis und/oder Topinambur und/oder Kartoffelstärke bzw. -flocken und/oder Kirsche enthalten sind, wobei zur Erhöhung der Hypoallergenität die Rohstoffe auch hydrolisiert, also aufgeschlossen sein können.

3. Ernährungsprodukt nach Anspruch 2, **dadurch gekennzeichnet, dass** das Reismilchpulver als sprühgetrocknetes Instantpulver vorliegt, wobei es auf einem Trägermaterial gebunden ist.

4. Ernährungsprodukt nach Anspruch 3, **dadurch gekennzeichnet, dass** das Reismilchpulver 10 - 80 Teile Stärke, vorzugsweise Kartoffelstärke, als Trägermaterial enthält.

5. Ernährungsprodukt nach einem der Ansprüche 1 - 2, **dadurch gekennzeichnet, dass** es als Fertiggetränk oder Fertigeis vorliegt.

6. Ernährungsprodukt nach einem der Ansprüche 1 - 5, **dadurch gekennzeichnet, dass** als Gamma-Linolensäurehaltige Stoffe Borretschsamenöl und/oder schwarze Johannesbeerkerne enthalten sind.

7. Ernährungsprodukt nach einem der Ansprüche 1 - 6, **dadurch gekennzeichnet, dass** als Omega-3-Fettsäuren enthaltende Stoffe Leinsamen, Leinöl, schwarze Johannesbeerkerne, Fischöl, grünes Blattgemüse bzw. deren Extrakte, enthalten sind.

8. Ernährungsprodukt nach einem der Ansprüche 2 - 7, **dadurch gekennzeichnet, dass** das getrocknete Reismilchpulver mit Gamma-Linolensäure und Omega-3-Fettsäuren angereichert ist.

9. Ernährungsprodukt nach einem der vorhergehenden Ansprüche, **dadurch gekennzeichnet, dass** es als Zusatzstoff wenigstens eine Darmflora und/oder Darmschleimhaut kräftigende Substanz enthält.

10. Ernährungsprodukt nach Anspruch 9, **dadurch gekennzeichnet, dass** als Darmflora und/oder Darmschleimhaut kräftigende Substanz Inulin und/oder Oligofructose und/oder Apfelpektin und/oder Guar und/oder Carob enthalten ist oder sind.

11. Ernährungsprodukt nach Anspruch 9 oder 10, **dadurch gekennzeichnet, dass** als Darmflora und/oder Darmschleimhaut kräftigende Substanz Amaranth bzw. Kartoffeln enthalten ist oder sind.

12. Ernährungsprodukt nach einem der Ansprüche 1 bis 11, **dadurch gekennzeichnet, dass** ferner Vitamine, insbesondere die Vitamine B1, B2, B6, B12, C, E, und/oder Beta-Carotin Niacin, Pantothensäure, Folsäure und Biotine enthalten sind.

13. Ernährungsprodukt nach einem der Ansprüche 1 - 12, **dadurch gekennzeichnet, dass** ferner Spurenelemente, Jodsalz, Mineralstoffe wie Calcium, Magnesium oder Zink bzw. mineralstoffhaltige Substanzen wie Zinkhefe, Aromen und/oder Hilfsstoffe wie Backpulver oder Zitronensäure enthalten sind.

**Claims**

1. Nutritional product for the dietary support of the treatment of atopic dermatitis, its basic substances consisting of hypoallergenic raw materials, **characterised in that** it contains as additives at least one gammalinoleic acid-containing material and an omega-3-fatty acid-containing material, the additive combination contributing to the balancing of metabolic disorders in atopic dermatitis and/or to the reduction of itching.

2. Nutritional product according to claim 1, **characterised in that** dried rice milk powder and/or kamut and/or amaranth and/or rice and/or Jerusalem artichoke and/or potato starch or flakes and/or cherry are contained as hypoallergenic raw materials, wherein the raw materials may also be hydrolysed, in other words may be pulped to increase the hypoallergenity.

3. Nutritional product according to claim 2, **characterised in that** the rice milk powder is present as spray-dried instant powder, being bound on a carrier material.

4. Nutritional product according to claim 3, **characterised in that** the rice milk powder contains 10 to 80 parts starch, preferably potato starch, as carrier material.

5. Nutritional product according to either of claims 1 to 2, **characterised in that** it is present as a ready-to-serve drink or ready-to-serve ice cream.

6. Nutritional product according to any one of claims 1 to 5, **characterised in that** borage seed oil and/or blackcurrant pips are contained as gammalinoleic acid-containing materials.

7. Nutritional product according to any one of claims 1 to 6, **characterised in that** linseed, linseed oil, blackcurrant pips, fish oil, green leaf vegetables or their extracts are contained as omega-3-fatty acid-containing materials.

8. Nutritional product according to any one of claims 2 to 7, **characterised in that** the dried rice milk powder is enriched with gammalinoleic acid and omega-3-fatty acids.

9. Nutritional product according to any one of the preceding claims, **characterised in that** it contains at least one substance which strengthens the intestinal flora and/or intestinal mucosa as an additive.

10. Nutritional product according to claim 9, **characterised in that** inulin and/or oligofructose and/or apple pectin and/or guar and/or carob is or are contained as a substance which strengthens the intestinal flora and/or intestinal mucosa.

11. Nutritional product according to claim 9 or 10, **characterised in that** amaranth or potatoes is or are contained as a substance which strengthens the intestinal flora and/or intestinal mucosa.

12. Nutritional product according to any one of claims 1 to 11, **characterised in that** vitamins are also contained, in particular the vitamins B1, B2, B6, B12, C, E and/or betacarotene niacin, pantothenic acid, folic acid and biotin.

13. Nutritional product according to any one of claims 1 to 12, **characterised in that** trace elements, iodine salt, mineral materials such as calcium, magnesium or zinc or mineral material-containing substances such as zinc yeast, aromas and/or auxiliary agents such as baking powder or citric acid are also contained.

**Revendications**

1. Produit alimentaire pour soutenir, d'un point de vue diététique, le traitement de la dermatite atopique, dans lequel les substances de base sont constituées de matières premières hypoallergéniques, **caractérisé en ce qu'**il comprend, en tant qu'additifs, au moins une substance contenant de l'acide gamma-linolénique et une substance contenant des acides gras oméga-3, moyennant quoi la combinaison d'additifs contribue à neutraliser les troubles métaboliques en cas de dermatite atopique et/ou à atténuer le prurit.

**2.** Produit alimentaire selon la revendication 1, **caractérisé en ce qu'**il contient, en tant que matières premières hypoallergéniques, de la poudre de lait de riz séché et/ou du kamut et/ou de l'amarante et/ou du riz et/ou du topinambour et/ou de l'amidon ou des flocons de pomme de terre et/ou de la cerise, moyennant quoi pour augmenter l'effet hypoallergénique, les matières premières peuvent également faire l'objet d'une réaction d'hydrolyse, à savoir être désagrégées.

**3.** Produit alimentaire selon la revendication 2, **caractérisé en ce que** la poudre de lait de riz se présente sous la forme d'une poudre de préparation pour boissons instantanées séchée par pulvérisation, moyennant quoi il est lié à un substrat.

**4.** Produit alimentaire selon la revendication 3, **caractérisé en ce que** la poudre de lait de riz présente 10 à 80 parties d'amidon, de préférence de l'amidon de pomme de terre, en tant que substrat.

**5.** Produit alimentaire selon l'une des revendications 1 ou 2, **caractérisé en ce qu'**il est utilisé sous la forme de glace ou boisson prête à consommer.

**6.** Produit alimentaire selon l'une quelconque des revendications 1 à 5, **caractérisé en ce que**, en tant que substance contenant de l'acide gamma-linolénique, on trouve de l'huile de bourrache et/ou des pépins de cassis.

**7.** Produit alimentaire selon l'une quelconque des revendications 1 à 6, **caractérisé en ce que** les substances contenant des acides gras oméga-3 contiennent des graines de lin, de l'huile de lin, des graines de cassis, de l'huile de poisson, des légumes verts à feuilles ou leurs extraits.

**8.** Produit alimentaire selon l'une quelconque des revendications 2 à 7, **caractérisé en ce que** la poudre de lait de riz séché est enrichie avec de l'acide gamma-linolénique et d'acides gras oméga-3.

**9.** Produit alimentaire selon l'une quelconque des revendications précédentes, **caractérisé en ce qu'**il contient, en tant qu'additif, au moins une substance renforçant la flore ou la muqueuse intestinale.

**10.** Produit alimentaire selon la revendication 9, **caractérisé en ce que** la substance renforçant la flore et/ou la muqueuse intestinale est de l'inuline et/ou de l'oligofructose et/ou de la pectine de pomme et/ou des graines de guarée et/ou de caroube.

**11.** Produit alimentaire selon la revendication 9 ou 10, **caractérisé en ce qu'**il contient, en tant que substance renforçant la flore et/ou la muqueuse intestinale, de l'amarante ou des pommes de terre.

**12.** Produit alimentaire selon l'une quelconque des revendications 1 à 11, **caractérisé en ce qu'**il contient en outre des vitamines, notamment de la vitamine B1, B2, B6, B12, C, E et/ou de la niacine, du bêta-carotène, de l'acide pantothénique, du folate et de la biotine.

**13.** Produit alimentaire selon l'une quelconque des revendications 1 à 12, **caractérisé en ce qu'**il contient en outre des oligo-éléments, du sel iodé, des substances minérales comme le calcium, le magnésium ou le zinc ou des substances contenant des sels minéraux comme la levure au zinc, des arômes et/ou des adjuvants comme la levure chimique ou l'acide citrique.